# EUROPÄISCHE PATENTSCHRIFT

(11) **EP 1 713 764 B1**
(45) Veröffentlichungstag und Bekanntmachung des Hinweises auf die Patenterteilung: **29.08.2007**
(21) Anmeldenummer: 05701200.7
(22) Anmeldetag: 27.01.2005
(51) Int. Cl.: C07C 253/30, C07C 255/07

(54) **HOMOGENE ISOMERISIERUNG VON CIS-2-PENTENNITRIL ZU 3-PENTENNITRIL**
HOMOGENEOUS ISOMERIZATION OF CIS-2-PENTENE NITRILE TO FORM 3-PENTENE NITRILE
ISOMERISATION HOMOGENE DE CIS-2-PENTENE-NITRILE EN 3-PENTENE-NITRILE

(30) Priorität: 29.01.2004 DE 102004004717
(43) Veröffentlichungstag der Anmeldung: 25.10.2006
(73) Patentinhaber: BASF Aktiengesellschaft, 67056 Ludwigshafen (DE)
(72) Erfinder: SCHEIDEL, Jens, 69493 Hirschberg (DE); MILLER, Christian, 67152 Ruppertsberg (DE); BAUMANN, Robert, 68159 Mannheim (DE); JUNGKAMP, Tim, B-2950 Kapellen (BE); BARTSCH, Michael, 67433 Neustadt (DE); HADERLEIN, Gerd, 67269 Grünstadt (DE); LUYKEN, Hermann, 67069 Ludwigshafen (DE); SIEGEL, Wolfgang, 67117 Limburgerhof (DE); BASSLER, Peter, 68519 Viernheim (DE)
(86) Internationale Anmeldenummer: PCT/EP2005/000770
(87) Internationale Veröffentlichungsnummer: WO 2005/073176

(56) Entgegenhaltungen:
- EP-A- 1 191 018
- US-A- 3 526 654
- US-A- 3 564 040
- US-A- 3 852 325
- VON DOERING, W. ET AL: "CryptoCope Rearrangement of 1,3-Dicyano-5-phenyl-4,4-d2-hexa-2,5-diene . Chameleonic or Centauric ?" JOURNAL OF THE AMERICAN CHEMICAL SOCIETY , 121(47), 10967-10975 CODEN: JACSAT; ISSN: 0002-7863, 1999, XP002320829

## Beschreibung

Die vorliegende Erfindung betrifft ein Verfahren zur Isomerisierung von Pentennitril in einem Eduktstrom.

Adipodinitril ist ein wichtiges Ausgangsprodukt in der Nylonherstellung, das durch zweifache Hydrocyanierung von 1,3-Butadien erhalten wird. In der ersten Hydrocyanierung wird das 1,3-Butadien zu 3-Pentennitril hydrocyaniert, wobei als Nebenprodukte hauptsächlich cis-2-Pentennitril, 2-Methyl-3-butennitril, 2-Methyl-2-butennitril, C₉-Nitrile und Methylglutamitril erhalten werden. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert. Dabei kann das cis-2-Pentennitril - im Gegensatz zu 3-Pentennitril, beispielsweise trans-3-Pentennitril - in Gegenwart der Nickel(0) enthaltenden Katalysatoren nicht zu Adipodinitril hydrocyaniert werden. Hierdurch wird die Ausbeute der Adipodinitril-Synthese herabgesenkt.

Wünschenswert ist es demnach, cis-2-Pentennitril zu trans-3-Pentennitril zu isomerisieren, um dieses dann wieder in die Adipodinitril-Synthese zurückführen zu können.

US 3,526,654 offenbart die Isomerisierung von cis-2-Pentennitril zu trans-3-Pentennitril in Gegenwart von Siliziumdioxid, Aluminiumoxid oder Natrium-Calcium-Silikat, wobei die Katalysatoren in verschiedenen Modifikationen vorliegen. Die Isomerisierung wird in der Flüssig- oder Gasphase bei Temperaturen von 25 °C bis 500 °C durchgeführt. Dieses Verfahren ist aufgrund eines geringen Umsatzes und einer langen Isomerisierungsdauer unwirtschaftlich. Im Allgemeinen kann die Geschwindigkeit einer Isomerisierung durch eine Erhöhung der Reaktionstemperatur angehoben werden. Dieses ist in der vorliegenden Isomerisierung von cis-2-Pentennitril zu trans-3-Pentennitril aber nicht zweckdienlich, da im Fall von Pentennitrilen eine Erhöhung der Reaktionstemperatur innerhalb des in der US 3,526,654 offenbarten Temperaturbereichs zu einer Bildung einer technisch inakzeptablen hohen Menge an Oligomeren und Polymeren führt.

Die DE-A-103 23 803 beschreibt die Isomerisierung von cis-2-Pentennitril zu 3-Pentennitril an Aluminiumoxid als Katalysator. Hierbei werden im Allgemeinen Ausbeuten von 30 % bezüglich cis-2-Pentennitril erzielt. Wird der Umsatz an cis-2-Pentennitril erhöht, kommt es zu einer verstärkten Bildung von trans-2-Pentennitril gegenüber der gewünschten Bildung von trans-3-Pentennitril.

von Döring et al., JACS 1999, 121, 10967-10975 beschreibt die Equilibrierung von cis -2-Pentennitril mit DBN als Katalysator.

Der vorliegenden Erfindung liegt somit die Aufgabe zugrunde, ein Verfahren bereitzustellen, das die Isomerisierung von cis-2-Pentennitril zu trans-3-Pentennitril mit Umsätzen bezüglich des Isomerisierungsedukts ermöglicht, die wirtschaftlich vertretbar sind. Dabei soll gleichzeitig eine hohe Raum-Zeit-Ausbeute von trans-3-Pentennitril bezüglich cis-2-Pentennitril erreicht werden.

Die Aufgabe der vorliegenden Erfindung wird gelöst durch ein Verfahren zur Isomerisierung von Pentennitrilen in einem Eduktstrom.

Das erfindungsgemäße Verfahren ist dadurch gekennzeichnet, dass die Isomerisierung an mindestens einem im Anspruch 1 angegebenen homogen gelösten Katalysator stattfindet.

In der vorliegenden Erfindung wird cis-2-Pentennitril zu trans-3-Pentennitril isomerisiert

Bei einer Isomerisierung von cis-2-Pentennitril kann der Eduktstrom weitere Bestandteile enthalten, die insbesondere ausgewählt sind aus der Gruppe, bestehend aus C5-Mononitrilen, C6-Dinitrilen, aliphatischen C1- bis C16-Alkanen, cyclischen C1- bis C16-Alkanen, aliphatischen C1- bis C16-Alkenen, cyclischen C1- bis C16-Alkenen, besonders bevorzugt ausgewählt aus der Gruppe, bestehend aus trans-3-Pentennitril, trans-2-Pentennitril, cis-3-Pentennitril, 4-Pentennitril, Z-2-Methyl-2-butennitril, E-2-Methyl-2-butennitril, 2-Methyl-3-butennitril, Methylglutarsäuredinitril, Ethylbemsteinsäuredinitril, Adipodinitril, Valeriansäurenitril, Cyclohexan, Methylcyclohexan, n-Heptan, n-Octan, Vinylcyclohexan, Ethylidencyclohexen und Vinylcyclohexen.

Der Gehalt an cis-2-Pentennitril in dem Eduktstrom beträgt vorzugsweise 0,5 bis 100 Gew.-%, besonders bevorzugt 1,0 bis 98 Gew.-%, insbesondere 1,5 bis 97 Gew.-%.

Der in dem erfindungsgemäßen Verfahren eingesetzte Eduktstrom, der cis-2-Pentennitril enthält, fällt im Allgemeinen in an sich bekannten Verfahren an. Ein Beispiel hierfür ist ein Verfahren zur Hydrocyanierung von 3-Pentennitril, wobei unter 3-Pentennitril trans-3-Pentennitril, cis-3-Pentennitril, deren Gemische oder eine cis- bzw. trans-3-Pentennitril enthaltende Mischung verstanden wird. Alternativ kann der Eduktstrom aus einer Hydrocyanierung von 4-Pentennitril oder 4-Pentennitril enthaltenden Mischungen zu Adipodinitril stammen.

In einer bevorzugten Ausführungsform kann das erfindungsgemäße Verfahren in ein Hydrocyanierungsverfahren zur Herstellung von Adipodinitril integriert werden.

### Ausführungsform I

Die erfindungsgemäße Isomerisierung kann in einer ersten bevorzugten Ausführungsform in jeder geeigneten dem Fachmann bekannten Apparatur durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, bevorzugt Rohrreaktoren, jeweils gegebenenfalls mit Vorrichtungen zur Wärmeübertragung. Die Reaktion kann in mehreren, wie zwei oder drei, Apparaten durchgeführt werden.

Nach der Reaktion wird der Reaktionsaustrag vorzugsweise destillativ aufgearbeitet.

Diese Destillation kann in jeder geeigneten, dem Fachmann bekannten Vorrichtung durchgeführt werden. Für die Destillation geeignet sind Vorrichtungen, wie sie beispielsweise in: Kirk-Othmer, Encyclopedia of Chemical Technology, 4. Ed., Vol. 8, John Wiley & Sons, New York, 1996, Seiten 334 bis 338 beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann dabei in mehreren, wie zwei oder drei Vorrichtungen, durchgeführt werden. Die Destillation kann zudem einstufig im Sinne einer Teilverdampfung des Zulaufstroms erfolgen.

Dabei wird im Sumpf ein an Isomerisierungsprodukt, vorzugsweise 3-Pentennitril, gegenüber dem Reaktionsaustrag angereicherter Strom als Sumpfstrom und ein an Isomerisierungsprodukt, vorzugsweise 3-Pentennitril, gegenüber dem Reaktionsaustrag abgereicherter Strom als Kopfstrom erhalten. Der Sumpfstrom kann vorzugsweise einem Verfahren zur Hydrocyanierung von 3-Pentennitril zugeführt werden, wobei gegebenenfalls vorher verbliebener homogen gelöster Katalysator in geeigneter Weise, vor zugsweise destillativ, abgetrennt wird.

Demgemäß erfolgt die Durchführung des Verfahrens gemäß Ausführungsform I vorzugsweise in einer Apparateverschaltung, umfassend mindestens einen Reaktor und mindestens eine Destillationsvorrichtung, wobei die Reaktoren, falls mehr als ein Reaktor verwendet wird, unmittelbar hintereinander geschaltet sind und die Destillationsvorrichtungen, falls mehr als eine Destillationsvorrichtung verwendet wird, unmittelbar hintereinander geschaltet sind und wobei die mindestens eine Destillationsvorrichtung hinter dem mindestens einen Reaktor angeordnet ist.

Unter "unmittelbar hintereinander geschaltet" und "wobei die mindestens eine Destillationsvorrichtung hinter dem mindestens einen Reaktor angeordnet ist" wird im Sinne der vorliegenden Erfindung verstanden, dass die Reaktoren ohne Unterbrechung durch Destillationsvorrichtungen in Reihe geschaltet sind, so das der Isomerisierungsstrom erst nach dem Durchlaufen durch alle vorhanden Reaktoren in die erste Destillationsvorrichtung geführt wird.

### Ausführungsform II

Eine Möglichkeit zur Erhöhung des Umsatzes ist die Entfernung des Reaktionsproduktes der Isomerisierung, um somit das Gleichgewicht auf die Seite des gewünschten isomerisierten Pentennitrils zu verschieben. Eine Möglichkeit, das isomerisierte Pentennitril aus dem Gleichgewicht zu entfernen, besteht darin, den höheren Siedepunkt des isomerisierten Pentennitrils im Vergleich zu dem zu isomerisierenden Pentennitril auszunutzen. Daraus ergibt sich zunächst eine zweite bevorzugte Ausführungsform.

In dieser zweiten bevorzugten Ausführungsform werden mindestens zwei Apparateverschaltungen gemäß Ausführungsform I so in Reihe geschaltet, dass der Kopfstrom der Destillationsvorrichtung, der an Isomerisierungsprodukt, vorzugsweise 3-Pentennitril abgereichert wurde, als Eduktstrom der in der Kaskade folgenden Apparateverschaltung gemäß Ausführungsform I verwendet wird. Die Sumpfströme können getrennt oder gemeinsam gegebenenfalls von verbliebenem homogen gelösten Katalysator in geeigneter Weise, vorzugsweise destillativ, befreit und anschließend zum Beispiel einem Verfahren zur Hydrocyanierung von 3-Pentennitril zugeführt werden. Besonders bevorzugt ist es, die Sumpfströme gemeinsam aufzuarbeiten und dabei noch nicht umgesetztes cis-2-Pentennitril ebenfalls abzutrennen und wieder als Eduktstrom in die Kaskade einzuführen. Der Katalysatorstrom kann vollständig in den Reaktor der ersten Apparateverschaltungen gemäß Ausführungsform I geführt werden oder aufgeteilt und in Teilen jeweils in die Reaktoren der in Reihe geschalteten Apparateverschaltungen gemäß Ausführungsform I geführt werden.

Das Verfahren gemäß Ausführungsform II erfolgt somit vorzugsweise in mehr als einer Apparateverschaltung, wobei die einzelnen Apparateverschaltungen hintereinander in Reihe angeordnet sind und die einzelnen Apparateverschaltungen mindestens einen Reaktor und mindestens eine Destillationsvorrichtung umfassen, wobei die Reaktoren der einzelnen Apparateverschaltungen, falls mehr als ein Reaktor in der jeweiligen Apparateverschaltung verwendet wird, unmittelbar hintereinander geschaltet sind und die Destillationsvorrichtungen der einzelnen Apparateverschaltungen, falls mehr als eine Destillationsvorrichtung in der jeweiligen Apparateverschaltung verwendet wird, unmittelbar hintereinander geschaltet sind und wobei die mindestens eine Destillationsvorrichtung hinter dem mindestens einen Reaktor in der jeweiligen Apparateverschaltung angeordnet ist.

Der Katalysatorstrom in den Ausführungsformen I und II kann aus frisch eingesetztem Katalysator bestehen und gegebenenfalls rückgeführten Katalysator enthalten, der bei der Abtrennung aus dem mindestens einen Sumpfstrom anfällt.

### Ausführungsform III

Eine weitere Möglichkeit zur Erhöhung des Umsatzes durch die Entfernung des Reaktionsproduktes der Isomerisierung aus dem Gleichgewicht stellt die Ausführungsform III dar.

Das erfindungsgemäße Verfahren kann gemäß der dritten bevorzugten Ausführungsform in einer Destillationskolonne, mindestens umfassend eine Sumpfzone, eine Reaktionszone und eine Kopfzone, durchgeführt werden. Dabei sind Sumpfzone, Reaktionszone und Kopfzone in der vorgegebenen Reihenfolge von unten nach oben in der Destillationskolonne angeordnet. Es ist dabei nicht ausgeschlossen, dass auch in der Sumpf- oder Kopfzone noch Reaktion stattfindet.

Falls die Isomerisierung in einer Destillationskolonne durchgeführt wird, so kann die Destillationskolonne zusätzlich Einbauten mit destillativer Trennwirkung umfassen. Diese zusätzlichen Einbauten sind vorzugsweise oberhalb der Reaktionszone angeordnet. In der oberen Trennzone, d.h. der Trennzone oberhalb der Reaktionszone, werden leicht siedende Nebenkomponenten weitgehend von schwer siedenden Komponenten abgetrennt. So wird hier beispielsweise gegebenenfalls mit dem Eduktstrom eingetragenes E-2-Methyl-2-butennitril von trans-3-Pentennitril und trans-2-Pentennitril getrennt. Ebenso kann aus nicht isomerisiertem cis-2-Pentennitril trans-3-Pentennitril und trans-2-Pentennitril abgereichert werden.

Durch die Trennwirkung der Einbauten in der Reaktionszone wird das schwer siedende lsomerisierungsprodukt weitgehend von leicht siedenden Komponenten abgetrennt. So werden z.B. trans-2-Pentennitril und trans-3-Pentennitril von nicht umgesetztem cis-2-Pentennitril getrennt.

Diese Trennungen sind nur beispielhaft aufgeführt und sind nicht einschränkend.
Die Aufteilung der Kolonne in eine rein destillative Trennzone und eine Reaktionszone ist bestimmt durch die Zulaufstelle des den Katalysator enthaltenden Mediums und das Verdampfungsverhalten des Katalysators bei den vorherrschenden Druck- und Temperaturbedingungen.

Bei optimaler Kolonnenkonfiguration kann das gesamte cis-2-Pentennitril des Eduktstroms ohne zusätzlichen Reaktor umgesetzt werden und das gesamte trans-3-Pentennitril im Sumpf ohne zusätzlichen Trennapparat erhalten werden. Dabei sind die zusätzlichen Einbauten mit destillativer Trennwirkung (Trennzone) im Allgemeinen von Vorteil, aber nicht zwingend notwendig.

Trenn- und Reaktionszone der gegebenenfalls verwendeten Destillationskolonne bestehen im Allgemeinen aus mehreren unterschiedlichen Teilbereichen mit unterschiedlichen Funktionen. Die Teilbereiche unterscheiden sich durch die Aufgabe des Transportes von Gas zum Kopf der Kolonne und die Aufgabe des Ableitens von Flüssigkeit in Richtung des Kolonnensumpfes. Zudem können Flüssigkeitsverteiler innerhalb der Reaktionszone notwendig sein, um eine optimale Verteilung von Flüssigkeit über den Kolonnenquerschnitt zu gewährleisten. Auch Einbauten zum Eintragen von Wärme in die Kolonne können sich in der Reaktionszone befinden.

Zum Erzielen der destillativen Trennwirkung der Destillationskolonne werden Einbauten mit destillativer Trennwirkung verwendet. Als Einbauten für die Destillationskolonnen werden vorzugsweise strukturierte Blechpackungen, strukturierte Gewebepackungen, Glockenbödenen, Dual-flow-Böden oder Schüttungen aus Füllkörpern oder Kombinationen von zwei oder mehreren dieser Klassen von trennwirksamen Einbauten verwendet.

Vorzugsweise werden auch Kolonneneinbauten mit hoher Trennstufenzahl wie Metallgewebepackungen oder Blechpackungen mit geordneter Struktur, beispielsweise Sulzer MELAPAK, Sulzer BX, Montz B1-Typen oder Montz A3-Typen, verwendet. Zur Durchführung des erfindungsgemäßen Verfahrens verwendet man vorzugsweise Destillationskolonnen, die, umfassend die Reaktions- und Trennzonen, 10 bis 100 Böden, besonders bevorzugt 10 bis 60 Böden, aufweisen. Entsprechendes gilt für die sogenannten theoretischen Böden bei anderen Kolonneneinbauten.

Die Dimension der Reaktionszone der Destillationskolonne richtet sich nach dem angestrebten Umsatzgrad und der Menge an cis-2-Pentennitril im Eduktstrom. Der Zulauf des den Katalysator enthaltenden Mediums erfolgt vorzugsweise 10, besonders bevorzugt 5 theoretische Destillationsstufen unterhalb des Kopfabzugs, insbesondere über die Zulaufstelle, an der auch der Rücklauf auf die Kolonne geführt wird.

Der Katalysator kann mit oder getrennt vom Eduktstrom auf die Kolonne gegeben werden.

Der oder die Eduktströme, hier nur als der Eduktstrom bezeichnet, können über verschiedene Zulaufstellen der Kolonne zugeführt werden.

In der Destillationskolonne werden Druck und Temperatur vorzugsweise so eingestellt, dass sich hohe Reaktionsgeschwindigkeiten bei ausreichend hoher Selektivität ergeben. Dabei wird der Druck in der Kopfzone vorzugsweise so eingestellt, dass die Temperatur in der Sumpfzone zwischen 30 und 300 °C, bevorzugt zwischen 40 und 250 °C, insbesondere zwischen 50 und 200 °C, beträgt. Die Verweilzeit in der Destillationskolonne beträgt vorzugsweise 1 Minute bis 10 Stunden, besonders bevorzugt 12 Minuten bis 3 Stunden.

Die optimalen Temperatur- und Druckverhältnisse werden im Allgemeinen durch die Einbindung der Isomerisierung in ein Verfahren, beispielsweise die zweifache Hydrocyanierung von 1,3-Butadien zu Adipodinitril, und die Arbeitstemperatur des Katalysators bestimmt. Die Einstellung des Drucks kann dabei mit einer Vakuumpumpe und/oder einer Druckregeleinrichtung geschehen, so dass die Druckverhältnisse den Anforderungen des Verfahrens angepasst sind.

Zur Erhöhung der Verweilzeit in der Reaktionszone ist es möglich, einen Teilstrom durch eine oder mehrere Seitenabzüge aus der Destillationskolonne durch einen oder mehrere Behälter zu leiten und gegebenenfalls mit Hilfe jeweils einer Pumpe die diese Behälter verlassenden Teilströme wieder in die Kolonne zurückzuführen. Die Behälter können dabei mit heterogenem Katalysator gefüllt werden. Dabei ist eine bevorzugte Ausführungsform dadurch gegeben, dass die Behälter beheizt werden. Die Temperatur in den Behältern sollte vorzugsweise der Temperatur der Flüssigphase auf dem Abzugsboden entsprechen.

Es hat sich ferner als vorteilhaft erwiesen, wenn die Wärmezufuhr in das Destillationssystem, bestehend aus der Destillationskolonne und gegebenenfalls dem Behälter oder den Behältern, nicht nur über den Verdampfer, sondern zusätzlich über außenliegende Wärmetauscher oder über sich direkt auf den Kolonneneinbauten befindliche Wärmetauscher erfolgt.

Es ist zudem möglich, aus der Kolonne an beliebigen Stellen über Seitenabzüge Teilströme abzuziehen. So ist es beispielsweise auch möglich, die Kolonne unter totalem Rücklauf zu betreiben und über einen Seitenabzug unterhalb der Katalysatorpackung aber oberhalb des Zulaufs Zwischensieder im Siedebereich zwischen dem zu isomerisierenden Pentennitril und dem isomerisierten Pentennitril auszuschleusen.

Falls in dem erfindungsgemäßen Verfahren eine Destillationskolonne verwendet wird, so reichert sich am Kopf der Kolonne nicht umgesetztes zu isomerisierendes Pentennitril und gegebenenfalls leichter als zu isomerisierendes Pentennitril siedende Komponenten aus dem Eduktstrom, gegebenenfalls zusammen mit leicht siedenden Nebenprodukten der Isomerisierung, an. In einer bevorzugten Ausführungsform der vorliegenden Erfindung wird dieser Kopfstrom über eine Leitung in einen Kondensator geführt, auskondensiert und über eine weitere Leitung ausgeschleust. Dabei kann vorzugsweise ein Teil des Kondensats wieder als Rücklauf in die Destillationskolonne gegeben werden. In einer bevorzugten Ausführungsform beträgt die Menge des Teiles des Kondensats, die wieder auf die Kolonne gegeben wird, mehr als 50 % des Kondensats, vorzugsweise mehr als 90 % des Kondensats. Auf diese Weise kann durch den internen Rücklauf in der Kolonne ein vorteilhaftes Konzentrationsprofil eingestellt werden.

Die erfindungsgemäße Isomerisierung wird an einem homogenen Katalysator durchgeführt, der ausgewählt ist aus der Gruppe der C1- bis C20-Mono- und Diamine, vorzugsweise der C4- bis C9-Diamine, besonders bevorzugt Hexylamin. Darüber hinaus kann als homogener Katalysator eine ionische Flüssigkeit, verwendet werden, die ausgewählt ist aus der Gruppe, bestehend aus Brönstedt-Säure-Addukten von organischen stickstoffhaltigen Substanzen.

In einer besonders bevorzugten Ausführungsform nach einer der Ausführungsformen I bis III kann das erfindungsgemäße Verfahren zur Isomerisierung in ein Gesamtverfahren integriert sein, in dem man
a) 3-Pentennitril oder eine Mischung, enthaltend 3-Pentennitril, in Gegenwart eines Nickel(0) enthaltenden Katalysators nach an sich bekannten Verfahren zu Adipodinitril hydrocyaniert unter Erhalt von cis-2-Pentennitril als Nebenprodukt,
b) von der Produktmischung cis-2-Pentennitril ganz oder teilweise gegebenenfalls mit anderen Stoffen aus der Hydrocyanierung zusammen abtrennt, beispielsweise durch Destillation,
c) cis-2-Pentennitril aus Schritt b) nach dem oben beschriebenen erfindungsgemäßen Verfahren gemäß einer der Ausführungsformen 1 bis III isomerisiert unter Erhalt mindestens eines Sumpfstroms, enthaltend trans-3-Pentennitril, gegebenenfalls weiteren Verbindungen, die ausgewählt sind aus der Gruppe, bestehend aus trans-2-Pentennitril, 4-Pentennitril und cis-3-Pentennitril, und eines Kopfstroms, enthaltend nicht isomerisiertes cis-2-Pentennitril und gegebenenfalls leichter als trans-3-Pentennitril siedenden Verbinungen, die ausgewählt sind aus der Gruppe, bestehend aus C5-Nitrilen, beispielsweise Z-2-Methyl-2-butennitril, E-2-Methyl-2-butennitril, 2-Methyl-3-butennitril, Valeriansäurenitril sowie andere aus der Hydrocyanierung stammende leichter als trans-3-Pentennitril siedende Komponenten,
d) von dem in Schritt c) erhaltenen Sumpfstrom gegebenenfalls enthaltenes cis-2-Pentennitril abtrennt, beispielsweise durch Destillation, und in Schritt c) zurückführt unter Erhalt eines Reststroms,
e) den in Schritt d) erhaltenen Reststrom, gegebenenfalls unter geeigneter Abtrennung des gegebenenfalls enthaltenen Isomerisierungskatalysators, vorzugsweise destillativ, ganz oder teilweise in Schritt a) zurückführt.

Der Sumpftstrom aus c) kann einen Restanteil an cis-2-Pentennitril enthalten. Dieser ist vorzugsweise kleiner 10 Gew.-%, besonders bevorzugt kleiner 1 Gew.-%, bezogen auf den Sumpfstrom.

Der Kopfstrom aus c) kann einen Restanteil trans-3-Pentennitril enthalten. Dieser ist vorzugsweise kleiner 10 Gew.%, besonders bevorzugt kleiner 5 Gew.-%, bezogen auf den Kopfstrom.

Vorzugsweise kann man in Schritt a) als Nickel(0) enthaltenden Katalysator einen solchen einsetzen, der neben Nickel(0) weiterhin einen einbindigen oder einen mehrbindigen oder ein Gemisch aus ein- und mehrbindigen Liganden, besonders bevorzugt einen einbindigen und einen Chelatliganden, insbesondere bevorzugt einen Chelatliganden, der mehrere, wie zwei oder drei, zur Bindung an das besagte Nickel(0) fähige dreibindige Phosphoratome, die unabhängig voneinander als Phosphin, Phosphinit, Phosphonit oder Phosphit vorliegen können, aufweist. Besonders vorteilhaft sollte der Katalysator weiterhin eine Lewissäure enthalten. Derartige Katalysatorsysteme sind an sich bekannt.

### Die vorliegende Erfindung wird anhand der folgenden Beispiele näher erläutert:

### Ausführungsbeispiel:

Homogen katalysierte Isomerisierung von cis-2-Pentennitril in einem Gemisch mit trans-3-Pentennitril.

Die nachfolgenden Beispiele 1 und 2 sollen die Isomerisierbarkeit mit Zusatz einer homogen gelösten Substanz verdeutlichen.

### Beispiel 1:

### Durchführung:

In einem Dreihalskolben werden 30 g cis-2-Pentennitril (gaschromatographische Analyse in Flächen-%: 98,74 % cis-Pentennitril, 0,64 % Z-2-Methyl-2-butennitril, 0,40 % trans-2-Pentennitril, 0,22 % 4-Pentrinnitril) vorgelegt. Dem cis-2-Pentennitril wird anschließend eine definierte Menge Hexylamin zugesetzt. Über einen Hals wird ein Thermometer in den Kolben geführt, auf den mittleren Hals wird ein Rückflusskühler aufgesetzt und am dritten Hals wird der Kolben mit einem Septum zur Probennahme während des Versuches verschlossen. Die Apparatur wird vor und während des Versuchs mit Argon gespült. Der Kolben wird nach dem Verschließen in einem Ölbad auf eine Innentemperatur von 100 °C temperiert. In regelmäßigen Abständen werden Proben über das Septum mit einer Spritze gezogen und mittels Gaschromatographie analysiert. Zu Versuchsbeginn liegt eine klare farblose Phase vor, nach Versuchsende liegt immer noch eine klare Phase vor, die nun leicht gelb gefärbt ist. Hexylamin ist auf der im Gaschromatographen verwendeten Stabilwax-Säule nicht gängig, weshalb die Nitrile nahezu 100 % der GC-Peaks verursachen. Alle Komponenten mit höheren Retentionszeiten als trans-3-Pentennitril werden unter der Komponente Hochsieder zusammengefasst.

In diesem Beispiel wird der Versuch mit Zugabe von 6,0 g Hexylamin durchgeführt.

### Verwendete Abkürzungen:

| | |
|---|---|
| Z2M2BN: | Z-2-Methyl-2-butennitril |
| C2PN: | cis-2-Pentennitril |
| T2PN: | trans-2-Pentennitril |
| 4PN: | 4-Pentennitril |
| T3PN: | trans-3-Pentennitril. |

### Ergebnis

| **Laufzeit** | **Z2M2BN** | **C2PN** | **T2PN** | **4PN** | **T3PN** | **Hochsieder** |
|---|---|---|---|---|---|---|
| 0 h | 0,64 % | 98,74 % | 0,40 % | 0,22 % | 0,00 % | 0,00 % |
| 2 h | 0,64 % | 93,01 % | 1,02 % | 4,14 % | 0,82 % | 0,37 % |
| 4 h | 0,64 % | 88,09 % | 2,03 % | 7,36 % | 1,18 % | 0,71 % |
| 6 h | 0,64 % | 83,65 % | 3,24 % | 10,09 % | 1,36 % | 1,02 % |

### Beispiel 2:

### Durchführung:

Wie in Beispiel 1 beschrieben.

In diesem Beispiel wird der Versuch mit Zugabe von 15,0 g Hexylamin durchgeführt.

### Ergebnis

| **Laufzeit** | **Z2M2BN** | **C2PN** | **T2PN** | **4PN** | **T3PN** | **Hochsieder** |
|---|---|---|---|---|---|---|
| 0 h | 0,63 % | 98,10 % | 0,46 % | 0,53 % | 0,00 % | 0,00 % |
| 2h | 0,62 % | 80,63 % | 4,15 % | 11,42 % | 1,30 % | 1,58 % |
| 4 h | 0,61 % | 70,42 % | 7,88 % | 16,45 % | 1,53 % | 3,13 % |
| 6 h | 0,61 % | 62,77 % | 11,51 % | 18,97 % | 1,60 % | 4,53 % |

Die Beispiele 1 und 2 zeigen, dass aufgrund des Zusatzes an Hexylamin die Bildung von Isomeren des cis-2-Pentennitrils stattfindet. Dabei ist Hexylamin homogen gelöst.

## Patentansprüche

1. Verfahren zur Isomerisierung von Pentennitril in einem Eduktstrom, **dadurch gekennzeichnet, dass** die Isomerisierung von cis-2-Pentennitril zu trans-3-Pentennitril an mindestens einem in dem Eduktstrom homogen gelösten Katalysator stattfindet, der ein C₁- bis C₂₀-Mono- oder -Diamin, oder eine ionische Flüssigkeit ist, wobei die ionische Flüssigkeit ausgewählt wird aus der Gruppe, bestehend aus Brönstedt-Säure-Adukten von organischen stickstoffhaltigen Substanzen.

2. Verfahren nach Anspruch 1, **dadurch gekennzeichnet, dass** das Verfahren in einer Apparateverschaltung, umfassend mindestens einen Reaktor und mindestens eine Destillationsvorrichtung, durchgeführt wird, wobei die Reaktoren, falls mehr als ein Reaktor verwendet wird, unmittelbar hintereinander geschaltet sind und die Destillationsvorrichtungen, falls mehr als eine Destillationsvorrichtung verwendet wird, unmittelbar hintereinander geschaltet sind und wobei die mindestens eine Destillationsvorrichtung hinter dem mindestens einen Reaktor angeordnet ist.

3. Verfahren nach Anspruch 1, **dadurch gekennzeichnet, dass** das Verfahren in mehr als einer Apparateverschaltung durchgeführt wird, wobei die einzelnen Apparateverschaltungen hintereinander in Reihe angeordnet sind und die einzelnen Apparateverschaltungen mindestens einen Reaktor und mindestens eine Destillationsvorrichtung umfassen, wobei die Reaktoren der einzelnen Apparatever schaltungen, falls mehr als ein Reaktor in der jeweiligen Apparateverschaltung verwendet wird, unmittelbar hintereinander geschaltet sind und die Destillationsvorrichtungen der einzelnen Apparateverschaltungen, falls mehr als eine Destillationsvorrichtung in der jeweiligen Apparateverschaltung verwendet wird, unmittelbar hintereinander geschaltet sind und wobei die mindestens eine Destillationsvorrichtung hinter dem mindestens einen Reaktor in der jeweiligen Apparateverschaltung angeordnet ist.

4. Verfahren nach Anspruch 1, **dadurch gekennzeichnet, dass** die Isomerisierung in einer Destillationskolonne durchgeführt wird.

5. Verfahren nach einem der Ansprüche 1 bis 4, **dadurch gekennzeichnet, dass** der Eduktstrom weitere Komponenten, ausgewählt aus der Gruppe, bestehend aus C5-Mononitrilen, C6-Dinitrilen, aliphatischen C1- bis C16-Alkanen, cyclischen C1- bis C16-Alkanen, aliphatischen C1- bis C16-Alkenen, cyclischen C1- bis C16-Alkenen, enthält.

6. Verfahren nach einem der Ansprüche 1 bis 5, **dadurch gekennzeichnet, dass** der Eduktstrom aus einem Verfahren zur Hydrocyanierung von 3-Pentennitril stammt.

## Claims

1. A process for isomerizing pentenenitrile in a reactant stream, wherein the isomerization of cis-2-pentenenitrile to trans-3-pentenenitrile takes place over at least one catalyst which is dissolved homogeneously in the reactant stream and is a C₁-to C₂₀-mono- or -diamine or an ionic liquid, the ionic liquid being selected from the group consisting of Brønsted acid adducts of organic nitrogen-containing substances.

2. The process according to claim 1, which is carried out in an apparatus connection comprising at least one reactor and at least one distillation apparatus, the reactors, if more than one reactor is used, being connected directly in series, and the distillation apparatuses, if more than one distillation apparatus is used, being connected directly in series, and the at least one distillation apparatus being connected downstream of the at least one reactor.

3. The process according to claim 1, which is carried out in more than one apparatus connection, the individual apparatus connections being connected in series and the individual apparatus connections comprising at least one reactor and at least one distillation apparatus, the reactors of the individual apparatus connections, if more than one reactor is used in the apparatus connection, being connected directly in series, and the distillation apparatuses of the individual apparatus connections, if more than one distillation apparatus is used in the particular apparatus connection, being connected directly in series, and the at least one distillation apparatus being connected downstream of the at least one reactor in the particular apparatus connection.

4. The process according to claim 1, wherein the isomerization is carried out in a distillation column.

5. The process according to any of claims 1 to 4, wherein the reactant stream comprises further components selected from a group consisting of C5-mononitriles, C6-dinitriles, aliphatic C1- to C16-alkanes, cyclic C1- to C16-alkanes, aliphatic C1- to C16-alkenes, cyclic C1- to C16-alkenes.

6. The process according to any of claims 1 to 5, wherein the reactant stream stems of a process for hydrocyanating 3-pentenenitrile.

## Revendications

1. Procédé d'isomérisation de pentènenitrile dans un courant de matière de départ, **caractérisé en ce que** l'isomérisation de cis-2-pentènenitrile en trans-3-pentènenitrile a lieu sur au moins un catalyseur qui est dissous de manière homogène dans le courant de matière de départ et qui est une monoamine ou diamine en C₁ à C₂₀ ou un liquide ionique, le liquide ionique étant choisi parmi le groupe constitué de produits d'addition d'acide de Brönsted de substances azotées organiques.

2. Procédé suivant la revendication 1, **caractérisé en ce que** le procédé est effectué dans un montage d'appareillage comprenant au moins un réacteur et au moins un dispositif de distillation, les réacteurs étant montés directement l'un derrière l'autre, dans le cas où on utilise plus d'un réacteur, et les dispositifs de distillation étant montés directement l'un derrière l'autre, dans le cas où on utilise plus d'un dispositif de distillation, ledit au moins un dispositif de distillation étant agencé derrière ledit au moins un réacteur.

3. Procédé suivant la revendication 1, **caractérisé en ce que** le procédé est effectué dans plus d'un montage d'appareillage, les différents montages d'appareillages étant agencés en série l'un derrière l'autre et chaque montage d'appareillage comportant au moins un réacteur et au moins un dispositif de distillation, les réacteurs des différents montages d'appareillage étant montés directement l'un derrière l'autre, dans le cas où on utilise plus d'un réacteur dans le montage d'appareillage respectif, et les dispositifs de distillation des différents montages d'appareillage étant montés directement l'un derrière l'autre, dans le cas où on utilise plus d'un dispositif de distillation dans le montage d'appareillage respectif, ledit au moins un dispositif de distillation étant agencé derrière ledit au moins un réacteur dans le montage d'appareillage respectif.

4. Procédé suivant la revendication 1, **caractérisé en ce que** l'isomérisation est effectuée dans une colonne de distillation.

5. Procédé suivant l'une des revendications 1 à 4, **caractérisé en ce que** le courant de matière de départ contient d'autres composants choisis parmi le groupe constitué des mononitriles en C₅, des dinitriles en C₆, des alcanes aliphatiques en C₁ à C₁₆, des alcanes cycliques en C₁ à C₁₆, des alcènes aliphatiques en C₁ à C₁₆, des alcènes cycliques en C₁ à C₁₆.

6. Procédé suivant l'une des revendications 1 à 5, **caractérisé en ce que** le courant de matière de départ provient d'un procédé d'hydrocyanatian de 3-pentènenitrile.
